# EUROPEAN PATENT SPECIFICATION

(11) **EP 2 179 056 B1**
(45) Date of publication and mention of the grant of the patent: **19.09.2012**
(21) Application number: 08785426.1
(22) Date of filing: 07.08.2008
(51) Int. Cl.: C12Q 1/68

(54) **EGFR INHIBITOR TREATMENT MARKER**
EGFR-INHIBITOR-BEHANDLUNGSMARKER
MARQUEUR DE TRAITEMENT PAR UN INHIBITEUR D'EGFR

(30) Priority: 14.08.2007 EP 07114312; 20.05.2008 EP 08156511
(43) Date of publication of application: 28.04.2010
(73) Proprietor: F. Hoffmann-La Roche AG, 4070 Basel (CH)
(72) Inventor: DELMAR, Paul, CH-4057 Basel (CH); KLUGHAMMER, Barbara, 79618 Rheinfelden (DE); LUTZ, Verena, 81371 Muenchen (DE); MCLOUGHLIN, Patricia, CH-4057 Basel (CH)
(74) Representative: Halbig, Dirk
(86) International application number: PCT/EP2008/006520
(87) International publication number: WO 2009/021681

(56) References cited:
- WO-A-2004/111273
- WO-A-2005/049829
- KAKIUCHI SOJI ET AL: "Prediction of sensitivity of advanced non-small cell lung cancers to gefitinib (Iressa, ZD1839)" HUMAN MOLECULAR GENETICS, OXFORD UNIVERSITY PRESS, SURREY, vol. 13, no. 24, 15 December 2004 (2004-12-15), pages 3029-3043, XP002440000 ISSN: 0964-6906
- COLDRENT C D ET AL: "Baseline Gene Expression Predicts Sensitivity to Gefitinib in Non-Small Cell Lung Cancer Cell Lines" MOLECULAR CANCER RESEARCH, AMERICAN ASSOCIATION FOR CANCER RESEARCH (A AC R), US, vol. 4, 1 July 2006 (2006-07-01), pages 521-528, XP003005576 ISSN: 1541-7786
- OKANO TETSUYA ET AL: "Proteomic signature corresponding to the response to gefitinib (Iressa, ZD1839), an epidermal growth factor receptor tyrosine kinase inhibitor in lung adenocarcinoma" CLINICAL CANCER RESEARCH, THE AMERICAN ASSOCIATION FOR CANCER RESEARCH, US, vol. 13, no. 3, 1 February 2007 (2007-02-01), pages 799-805, XP002440001 ISSN: 1078-0432
- KOKUBO Y ET AL: "Reduction of PTEN protein and loss of epidermal growth factor receptor gene mutation in lung cancer with natural resistance to gefitinib (IRESSA)." BRITISH JOURNAL OF CANCER 9 MAY 2005, vol. 92, no. 9, 9 May 2005 (2005-05-09), pages 1711-1719, XP007905848 ISSN: 0007-0920
- ZHOU BIN-BING S ET AL: "Targeting ADAM-mediated ligand cleavage to inhibit HER3 and EGFR pathways in non-small cell lung cancer" CANCER CELL, CELL PRESS, US, vol. 10, no. 1, 1 January 2006 (2006-01-01), pages 39-50, XP008093928 ISSN: 1535-6108
- GRIDELLI CESARE ET AL: "Erlotinib in non-small cell lung cancer treatment: current status and future development." THE ONCOLOGIST JUL 2007, vol. 12, no. 7, July 2007 (2007-07), pages 840-849, XP007905850 ISSN: 1083-7159

## Description

The present invention provides a biomarker that is predictive for the response to treatment with an erlotinib in NSCLC patients

A number of human malignancies are associated with aberrant or over-expression of the epidermal growth factor receptor (EGFR). EGF, transforming growth factor α (TGF-α), and a number of other ligands bind to the EGFR, stimulating autophosphorylation of the intracellular tyrosine kinase domain of the receptor. A variety of intracellular pathways are subsequently activated, and these downstream events result in tumour cell proliferation in vitro. It has been postulated that stimulation of tumour cells via the EGFR may be impor-tant for both tumour growth and tumour survival in vivo.

Early clinical data with Tarceva, an inhibitor of the EGFR tyrosine kinase, indicate that the compound is safe and generally well tolerated at doses that provide the targeted effective concentration (as determined by preclinical data). Clinical phase I and II trials in patients with advanced disease have demonstrated that Tarceva has promising clinical activity in a range of epithelial tumours. Indeed, Tarceva has been shown to be capable of inducing durable partial remissions in previously treated patients with head and neck can-cer, and NSCLC (Non small cell lung cancer) of a similar order to established second line chemotherapy, but with the added benefit of a better safety profile than chemo therapy and improved convenience (tablet instead of intravenous [i.v.] administration). A recently completed, randomised, double-blind, placebo-controlled trial (BR.21) has shown that single agent Tarceva significantly prolongs and improves the survival of NSCLC patients for whom standard therapy for advanced disease has failed.

Tarceva (erlotinib) is a small chemical molecule; it is an orally active, potent, selective inhibitor of the EGFR tyrosine kinase (EGFR-TKI).

Lung cancer is the major cause of cancer-related death in North America and Europe. In the United States, the number of deaths secondary to lung cancer exceeds the combined total deaths from the second (colon), third (breast), and fourth (prostate) leading causes of cancer deaths combined. About 75% to 80% of all lung cancers are non-small cell lung cancer (NSCLC), with approximately 40% of patients presenting with locally advanced and/or unresectable disease. This group typically includes those with bulky stage IIIA and IIIB disease, excluding malignant pleural effusions.

The crude incidence of lung cancer in the European Union is 52.5, the death rate 48.7 cases/100000/year. Among men the rates are 79.3 and 78.3, among women 21.6 and 20.5, respectively. NSCLC accounts for 80% of all lung cancer cases. About 90% of lung cancer mortality among men, and 80% among women, is attributable to smoking.

In the US, according to the American Cancer Society, during 2004, there were approximately 173,800 new cases of lung cancer (93,100 in men and 80,700 in women) and were accounting for about 13% of all new cancers. Most patients die as a consequence of their disease within two years of diagnosis. For many NSCLC patients, successful treat-ment remains elusive. Advanced tumours often are not amenable to surgery and may also be resistant to tolerable doses of radiotherapy and chemotherapy. In randomized trials the currently most active combination chemotherapies achieved response rates of approxi-mately 30% to 40% and a 1-year survival rate between 35% and 40%. This is really an ad-vance over the 10% 1-year survival rate seen with supportive care alone.

Until recently therapeutic options for patients following relapse were limited to best supportive care or palliation. A recent trial comparing docetaxel (Taxotere) with best supportive care showed that patients with NSCLC could benefit from second line chemother-apy after cisplatin-based first-line regimens had failed. Patients of all ages and with ECOG performance status of 0, 1, or 2 demonstrated improved survival with docetaxel, as did those who had been refractory to prior platinum-based treatment. Patients who did not benefit from therapy included those with weight loss of 10%, high lactate dehydrogenase levels, multi-organ involvement, or liver involvement. Additionally, the benefit of do-cetaxel monotherapy did not extend beyond the second line setting. Patients receiving do-cetaxel as third-line treatment or beyond showed no prolongation of survival. Single-agent docetaxel became a standard second-line therapy for NSCLC. Recently another randomized phase III trial in second line therapy of NSCLC compared pemetrexed (Alimta®) with do-cetaxel. Treatment with pemetrexed resulted in a clinically equivalent efficacy but with significantly fewer side effects compared with docetaxel.

It has long been acknowledged that there is a need to develop methods of individualising cancer treatment. With the development of targeted cancer treatments, there is a particular interest in methodologies which could provide a molecular profile of the tumour target, (i.e. those that are predictive for clinical benefit). Proof of principle for gene expres-sion profiling in NSCLC has already been established with the molecular classification of tumour types which are not apparent on the basis of current morphological and immunohistochemical tests.

Therefore, it is an aim of the present invention to provide expression biomarkers that are predictive for the response to erlotinib treatment in NSCLC patients.

In a first object the present invention provides an in vitro method of predicting the response of a NSCLC patient to treatment with an erlotinib comprising the steps:
determining an expression level of a gene PSPH in a tumour sample of a patient and com-paring the expression level of the gene PSPH to a value representative of the gene PSPH expression level in tumours of a non responding patient population, wherein a higher expression level of the gene PSPH in the tumour sample of the patient is indicative for a patient who will respond to the treatment, wherein benefiting population was defined as either having objective response, or disease stabilization for 12 weeks.

The term "a value representative of an expression level of the gene PSPH in tumours of a non responding patient population" refers to an estimate of the mean expression level of the marker gene in tumours of a population of patients who do not respond to the treatment with the erlotinib.

In a preferred embodiment, the marker gene PSPH (Seq. Id No. 1) shows typically between 1.8 and 3.7 ore more fold higher expression in the tumour sample of the responding patient compared to a value representative of the gene PSPH expression level in tu-mours of a non responding patient population.

In a preferred embodiment, the expression level of the gene PSPH is determined by microarray technology or other technologies that assess RNA expression levels like quantitative RT-PCR or by any method looking at the expression level of the respective protein, eg immunohistochemistry (IHC). The construction and use of gene chips are well known in the art, see, U. S. Pat Nos. 5,202,231; 5,445,934; 5,525,464; 5,695,940; 5,744,305; 5,795, 716 and 1 5,800,992. See also, Johnston, M. Curr. Biol. 8:R171-174 (1998); Iyer VR et al., Science 283:83-87 (1999). Of course, the gene expression level can be determined by other methods that are known to a person skilled in the art such as e.g. northern blots, RT-PCR, real time quantitative PCR, primer extension, RNase protection, RNA expression profiling.

The marker gene of the present invention can be combined with other biomarkers to biomarker sets. Biomarker sets can be built from any combination of predictive biomarkers to The marker gene used in the present invention can be combined with other biomarkers to biomarker sets. Biomarker sets can be built from any combination of predictive biomarkers to make predictions about the effect of erlotinib treatment in NSCLC patients. The biomarkers and biomarkers sets described herein can be used, for example, to predict how patients with NSCLC will respond to therapeutic intervention with erlotinib.

The term "gene" as used herein comprises variants of the gene. The term "variant" relates to nucleic acid sequences which are substantially similar to the nucleic acid sequences given by the GenBank accession number. The term "substantially similar" is well understood by a person skilled in the art. In particular, a gene variant may be an allele which shows nucleotide exchanges compared to the nucleic acid sequence of the most prevalent allele in the human population. Preferably, such a substantially similar nucleic acid sequence has a sequence similarity to the most prevalent allele of at least 80%, pref-erably at least 85%, more preferably at least 90%, most preferably at least 95%. The term "variants" is also meant to relate to splice variants.

Techniques for the detection of gene expression of the genes described by this invention include, but are not limited to northern blots, RT-PCR, real time quantitative PCR, primer extension, RNase protection, RNA expression profiling and related techniques. These techniques are well known to those of skill in the art see e.g. Sambrook J et al., Mo-lecular Cloning: A Laboratory Manual, Third Edition (Cold Spring Harbor Press, Cold Spring Harbor, 2000).

Techniques for the detection of protein expression of the respective genes described by this invention include, but are not limited to immunohistochemistry (IHC).

Cells from a patient tissue sample, e.g., a tumour or cancer biopsy, can be assayed to determine the expression pattern of one or more bio-markers. Success or failure of a cancer treatment can be determined based on the biomarker expression pattern of the cells from the test tissue (test cells), e.g., tumour or cancer biopsy, as being relatively similar or different from the expression pattern of a control set of the one or more biomarkers. In the context of this invention, it was found that the PSPH gene is up-regulated i.e. shows a higher expression level, in tumours of patients who respond to erlotinib treatment compared to tumours of patients who do not respond to erlotinib treatment. Thus, if the test cells show a biomarker expression profile which

corresponds to that of a patient who responded to NSCLC treatment, it is highly likely or predicted that the individual's NSCLC or tumour will respond favourably to treatment with erlotinib. By contrast, if the test cells show a biomarker expression pattern corresponding to that of a patient who did not respond to NSCLC treatment, it is highly likely or predicted that the individual's NSCLC or tumour will not respond to treatment with erlotinib.

The biomarker used in present invention is a first step towards an individualized therapy for patients with NSCLC, in particular patients with refractory NSCLC. This individualized therapy will allow treating physicians to select the most appropriate agent out of the existing drugs for NSCLC therapy. The benefit of individualized ther-apy for each future patient are: response rates / number of benefiting patients will increase and the risk of adverse side effects due to ineffective treatment will be reduced.

### Short description of the figures

Figure 1 shows the study design;
Figure 2 shows the scheme of sample processing.
Figure 3a shows expression levels versus clinical outcome for Genechip® profiling;
Figure 3b shows expression levels versus clinical outcome for qRT-PCR and
Figure 3c shows the correlation between Genechip® and qRT-PCR measurements for PSPH.

### Experimental part

### Rationale for the Study and Study Design

Recently mutations within the EGFR gene in the tumour tissue of a subset of NSCLC patients and the association of these mutations with sensitivity to erlotinib and gefitinib were described (Pao W, et al. 2004; Lynch et al. 2004; Paez et al. 2004). For the patients combined from two studies, mutated EGFR was observed in 13 of 14 patients who re-sponded to gefitinib and in none of the 11 gefitinib-treated patients who did not respond. The reported prevalence of these mutations was 8% (2 of 25) in unselected NSCLC pa-tients. These mutations were found more frequently in adenocarcinomas (21%), in tumours from females (20%), and in tumours from Japanese patients (26%). These mutations result in increased in vitro activity of EGFR and increased sensitivity to gefitinib. The relation-ship of the mutations to prolonged stable disease or survival duration has not been prospec-tively evaluated.

Based on exploratory analyses from the BR.21 study, it appeared unlikely that the observed survival benefit is only due to the EGFR mutations, since a significant survival benefit is maintained even when patients with objective response are excluded from analy-ses (data on file). Other molecular mechanisms must also contribute to the effect.

Based on the assumption that there are changes in gene expression levels that are predictive of response / benefit to Tarceva treatment, microarray analysis was used to detect these changes

This required a clearly defined study population treated with Tarceva monotherapy after failure of 1st line therapy. Based on the experience from the BR.21 study, benefiting population was defined as either having objective response, or disease stabilization for 12 weeks. Clinical and microarray datasets were analyzed according to a pre defined statistical plan.

The application of this technique requires fresh frozen tissue (FFT). Therefore a mandatory biopsy had to be performed before start of treatment. The collected material was frozen in liquid nitrogen (N2).

A second tumour sample was collected at the same time and stored in paraffin (formalin fixed paraffin embedded, FFPE). This sample was analysed for alterations in the EGFR signalling pathway.

The ability to perform tumour biopsies via bronchoscopy was a prerequisite for this study. Bronchoscopy is a standard procedure to confirm the diagnosis of lung cancer. Although generally safe, there is a remaining risk of complications, e.g. bleeding.

This study was a first step towards an individualized therapy for patients with refractory NSCLC. This individualized therapy will allow treating physicians to select the most appropriate agent out of the existing drugs for this indication.

Once individualized therapy will be available, the benefit for each future patient will outweigh the risk patients have to take in the present study:
response rates / number of benefiting patients will increase,
the risk of adverse side effects due to ineffective treatment will be reduced.
Rationale for Dosage Selection

Tarceva was given orally once per day at a dose of 150 mg until disease progres-sion, intolerable toxicities or death. The selection of this dose was based on pharmacoki-netic parameters, as well as the safety and tolerability profile of this dose observed in Phase I, II and III trials in heavily pre-treated patients with advanced cancer. Drug levels seen in the plasma of patients with cancer receiving the 150 mg/day dose were consistently above the average plasma concentration of 500 ng / ml targeted for clinical efficacy. BR.21 showed a survival benefit with this dose.

### Objectives of the Study

The primary objective was the identification of differentially expressed genes that are predictive for benefit (CR, PR or SD ? 12 weeks) of Tarceva treatment. Identification of differentially expressed genes predictive for "response" (CR, PR) to Tarceva treatment was an important additional objective.

The secondary objectives were to assess alterations in the EGFR signalling pathways with respect to benefit from treatment.

### Study Design

### Overview of Study Design and Dosing Regimen

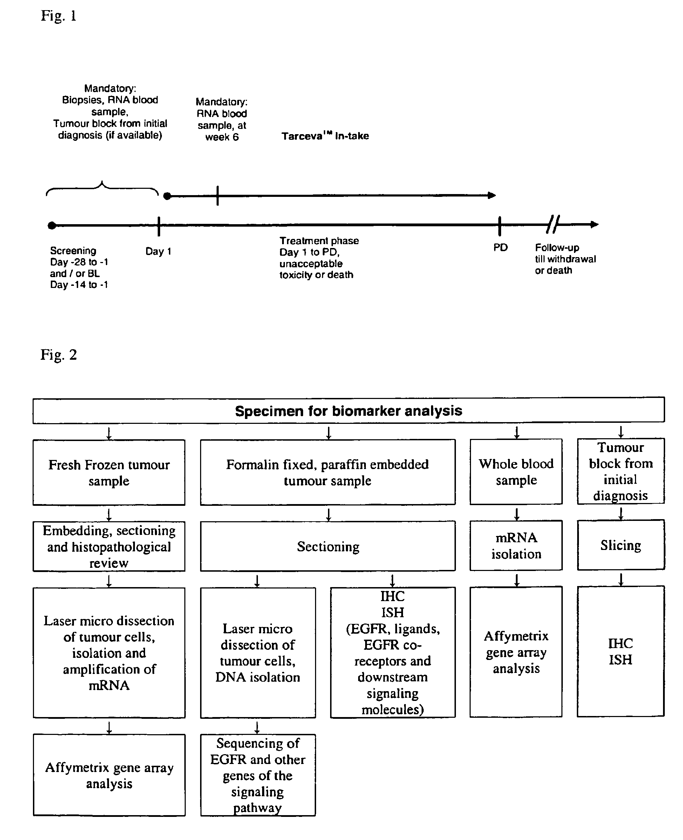

This was an open-label, predictive marker identification Phase II study. The study was conducted in approximately 26 sites in about 12 countries. 264 patients with advanced NSCLC following failure of at least one prior chemotherapy regimen were enrolled over a 12 month period. Continuous oral Tarceva was given at a dose of 150 mg/day. Dose re-ductions were permitted based on tolerability to drug therapy. Clinical and laboratory pa-rameters were assessed to evaluate disease control and toxicity. Treatment continued until disease progression, unacceptable toxicity or death. The study design is depicted in figure 1.

Tumour tissue and blood samples were obtained for molecular analyses to evaluate the effects of Tarceva and to identify subgroups of patients benefiting from therapy.

### Predictive Marker Assessments

Biopsies of the tumour were taken within 2 weeks before start of treatment. Two different samples were collected:
The first sample was always frozen immediately in liquid N2
The second sample was fixed in formalin and embedded in paraffin
Snap frozen tissue had the highest priority in this study.
Figure 2 shows a scheme of the sample processing.

### Microarray Analysis

The snap frozen samples were used for laser capture microdissection (LCM) of tu-mour cells to extract tumour RNA and RNA from tumour surrounding tissue. The RNA was analysed on Affymetrix microarray chips (HG-U133A) to establish the patients' tu-mour gene expression profile. Quality Control of Affymetrix chips was used to select those samples of adequate quality for statistical comparison.

### Single Biomarker Analyses on Formalin Fixed Paraffin Embedded Tissue

The second tumour biopsy, the FFPE sample, was used to perform DNA mutation, IHC and ISH analyses as described below. Similar analyses were performed on tissue col-lected at initial diagnosis.

The DNA mutation status of the genes encoding EGFR and other molecules involved in the EGFR signalling pathway were analysed by DNA sequencing. Gene amplification of EGFR and related genes were be studied by FISH.

Protein expression analyses included immunohistochemical [IHC] analyses of EGFR and other proteins within the EGFR signalling pathway.

### Response Assessments

The RECIST (Uni-dimensional Tumour Measurement) criteria were used to evaluate response. These criteria can be found under the following link: http://www.eortc.be/recist/

Note that:To be assigned a status of CR or PR, changes in tumour measurements must be confirmed by repeated assessments at least 4 weeks apart at any time during the treatment period.

In the case of SD, follow-up measurements must have met the SD criteria at least once after study entry at a minimum interval of 6 weeks.

In the case of maintained SD, follow-up measurements must have met the SD criteria at least once after study entry with maintenance duration of at least 12 weeks.

### Survival Assessment

A regular status check every 3 months was performed either by a patient's visit to the clinic or by telephone. All deaths were recorded. At the end of the study a definitive confirmation of survival was required for each patient.

### Response to erlotinib treatment

A total of 264 patients from 12 countries and 26 centres were enrolled in the study. 26% had Stage IIIB and 24% Stage IV NSCLC. 13.6% (n=36) of patients achieved an objective response while 31.4% (n=83) had clinical benefit (defined as having either an objective response or stable disease for 12 weeks or more). Median overall survival was 7.6 (CI 7-9) months and median progression-free survival was 11.3 (CI 8-12) weeks. Full de-tails about the clinical data are shown in Table 1.

Fresh frozen bronchoscopic biopsies were collected from all subjects, but either not all samples had sufficient tumour content prior to microdissection (LCM) or did not have sufficient RNA yield after LCM to proceed to microarray analysis, so that tumour material was only available for 125 patients; 122 of these had evaluable RNA. Another set of 20 samples did not pass our quality control assessment of the microarray data. Of the 102 microarray data sets that were suitable for statistical analysis, the clinical characteristics are shown in Table 1. While 36 patients in the overall study achieved an objective response, only 6 of these had microarray data; similarly for those achieving clinical benefit the num-ber of subjects with microarray data was only 21 as compared to 83 in the full data set. 6 were judged to be partial responders (PR), 31 had SD and 49 had PD; of the 6 patients with a PR, 5 had adenocarcinoma and one had squamous cell carcinoma. There were no patients achieving a CR in the data set.

### Methods

### RNA sample preparation and quality control of RNA samples

All biopsy sample processing was handled by a pathology reference laboratory; fresh frozen tissue samples were shipped from investigator sites to the Clinical Sample Opera-tions facility in Roche Basel and from there to the pathology laboratory for further process-ing. Laser capture microdissection was used to select tumour cells from surrounding tis-sue. After LCM, RNA was purified from the enriched tumour material. The pathology laboratory then carried out a number of steps to make an estimate of the concentration and quality of the RNA..

RNases are RNA degrading enzymes and are found everywhere and so all procedures where RNA will be used must be strictly controlled to minimize RNA degradation. Most mRNA species themselves have rather short half-lives and so are considered quite unstable. Therefore it is important to perform RNA integrity checks and quantification before any assay.

RNA concentration and quality profile can be assessed using an instrument from Agilent (Agilent Technologies, Inc., Palo Alto, CA) called a 2100 Bioanalyzer®. The instrument software generates an RNA Integrity Number (RIN), a quantitation estimate (Schroeder, A., et al., The RIN: an RNA integrity number for assigning integrity values to RNA measurements. BMC Mol Biol, 2006. 7: p. 3), and calculates ribosomal ratios of the total RNA sample. The RIN is determined from the entire electrophoretic trace of the RNA sampie, and so includes the presence or absence of degradation products.

The RNA quality was analysed by a 2100 Bioanalyzer®. Only samples with at least one rRNA peak above the added poly-I noise and sufficient RNA were selected for further analysis on the Affymetrix platform. The purified RNA was forwarded to the Roche Cen-tre for Medical Genomics (RCMG; Basel, Switzerland) for analysis by microarray. 122 RNA samples were received from the pathology laboratory for further processing.

### Target Labeling of tissue RNA samples

Target labeling was carried out according to the Two-Cycle Target Labeling Amplification Protocol from Affymetrix (Affymetrix, Santa Clara, California), as per the manufacturer's instructions.

The method is based on the standard Eberwine linear amplification procedure but uses two cycles of this procedure to generate sufficient labeled cRNA for hybridization to a microarray.

Total RNA input used in the labeling reaction was 10ng for those samples where more than 10ng RNA was available; if less than this amount was available or if there was no quantity data available (due to very low RNA concentration), half of the total sample was used in the reaction. Yields from the labeling reactions ranged from 20-180µg cRNA. A normalization step was introduced at the level of hybridization where 15µg cRNA was used for every sample.

Human Reference RNA (Stratagene, Carlsbad, CA, USA) was used as a control sample in the workflow with each batch of samples. 10ng of this RNA was used as input alongside the test samples to verify that the labeling and hybridization reagents were working as expected.

### Microarray hybridizations

Affymetrix HG-U133A microarrays contain over 22,000 probe sets targeting approximately 18,400 transcripts and variants which represent about 14,500 well-characterized genes.

Hybridization for all samples was carried out according to Affymetrix instructions (Affymetrix Inc., Expression Analysis Technical Manual, 2004). Briefly, for each sample, 15µg of biotin-labeled cRNA were fragmented in the presence of divalent cations and heat and hybridized overnight to Affymetrix HG-U133A full genome oligonucleotide arrays. The following day arrays were stained with streptavidin-phycoerythrin (Molecular Probes; Eugene, OR) according to the manufacturer's instructions. Arrays were then scanned using a GeneChip Scanner 3000 (Affymetrix), and signal intensities were automatically calcu-lated by GeneChip Operating Software (GCOS) Version 1.4 (Affymetrix).

### Statistical Analysis

Analysis of the Affymetrix™ data consisted of four main steps.

Step 1 was quality control. The goal was to identify and exclude from analysis array data with a sub-standard quality profile.

Step 2 was pre-processing and normalization. The goal was to create a normalized and scaled "analysis data set", amenable to inter-chip comparison. It comprised background noise estimation and subtraction, probe summarization and scaling.

Step 3 was exploration and description. The goal was to identify potential bias and sources of variability. It consisted of applying multivariate and univariate descriptive analysis techniques to identify influential covariates.

Step 4 was modeling and testing. The goal was to identify a list of candidate markers based on statistical evaluation of the difference in mean expression level between "Responders" (patients with "Partial Response" or "Complete Response" as best response) and "Non Responders" (patients "Progressive Disease" as best response). It consisted of fitting an adequate statistical model to each probe-set and deriving a measure of statistical signifi-cance. All analyses were performed using the R software package.

### Step 1 : Quality Control

The assessment of data quality was based on checking several parameters. These included standard Affymetrix GeneChip™ quality parameters, in particular: Scaling Factor, Percentage of Present Call, and Average Background. This step also included visual inspection of virtual chip images for detecting localized hybridization problems, and comparison of each chip to a virtual median chip for detecting any unusual departure from median behaviour. Inter-chip correlation analysis was also performed to detect outlier sam-ples. In addition, ancillary measures of RNA quality obtained from analysis of RNA sam-ples with the Agilent Bioanalyzer™ 2100 were taken into consideration.

Based on these parameters, data from 20 arrays were excluded from analysis. Thus data from a total of 102 arrays representing 102 patients was included in the analysis. The clinical description of these 102 patients set is reported in table 1.

**Table 1: Description of clinical characteristics of patients included in the analysis**

| **Variable** | **Value** | **n=102** |
|---|---|---|
| | | n (%) |
| Best Response | N/A | 16 (15.7%) |
| | PD | 49 (48.0%) |
| | SD | 31 (30.4%) |
| | PR | 6 (5.9%) |
| | | |
| Clinical Benefit | NO | 81 (79.4%) |
| | YES | 21 (20.6%) |
| | | |
| SEX | FEMALE | 25 (24.5%) |
| | MALE | 77 (74.5%) |
| | | |
| ETHNICITY | CAUCASIAN | 65 (63.7%) |
| | ORIENTAL | 37 (36.3%) |
| | | |
| Histology | ADENOCARCINOMA | 35 (34.3%) |
| | SQUAMOUS | 53 (52.0%) |
| | OTHERS | 14 (13.7%) |
| | | |
| Ever-Smoking | NO | 20 (19.6%) |
| | YES | 82 (80.4%) |

### Step 2 : Data pre-processing and normalization

The rma algorithm (Irizarry, R.A., et al., Summaries of Affymetrix GeneChip probe level data. Nucl. Acids Res., 2003. 31(4): p. e15) was used for pre-processing and normalization. The mas5 algorithm (AFFYMETRIX, GeneChip® Expression: Data Analysis Fundamentals. 2004, AFFYMETRIX) was used to make detection calls for the individual probe-sets. Probe-sets called "absent" or "marginal" in all samples were removed from further analysis; 5930 probe-sets were removed from analysis based on this criterion. The analysis data set therefore consisted of a matrix with 16353 (out of 22283) probe-sets measured in 102 patients.

### Step 3 : Data description and exploration

Descriptive exploratory analysis was performed to identify potential bias and major sources of variability. A set of covariates with a potential impact on gene expression pro-files was screened. It comprised both technical and clinical variables. Technical covariates included: date of RNA processing (later referred to as batch), RIN (as a measure of RNA quality/integrity), Operator and Center of sample collection. Clinical covariates included: histology type, smoking status, tumour grade, performance score (Oken, M.M., et al., Toxicity and response criteria of the Eastern Cooperative Oncology Group. Am J Clin Oncol, 1982. 5(6): p. 649-55), demographic data, responder status and clinical benefit status.

The analysis tools included univariate ANOVA and principal component analysis. For each of these covariates, univariate ANOVA was applied independently to each probe-set.

A significant effect of the batch variable was identified. In practice, the batch vari-able captured differences between dates of sample processing and Affymetrix chip lot. Af-ter checking that the batch variable was nearly independent from the variables of interest, the batch effect was corrected using the method described in Johnson, W.E., C. Li, and A. Rabinovic, Adjusting batch effects in microarray expression data using empirical Bayes methods. Biostat, 2007. 8(1): p. 118-127.

The normalized data set after batch effect correction served as the analysis data set in subsequent analyses.

Histology and RIN were two additional important variables highlighted by the descriptive analysis.

### Step 4 : Data modeling and testing.

A linear model was fitted independently to each probe-set. Variables included in the model are reported in table 2. The model parameters were estimated by the maximum likelihood technique. The parameter corresponding to the "Response" variable (X1) was used to assess the difference in expression level between the group "responding" and "non responding" patients.

**Table 2: Description of the variables included in the linear model. The linear model defined by these variables, including a normally distributed error term, was fitted to each probe-set.**

| **Variable** | **Type** | **Values** |
|---|---|---|
| **gene expression** | **Dependent (Yᵢₚ)** | **Normalized log2 intensity of probe-set i in patient p.** |
| Intercept | Overall mean (µ) | |
| **Response** | **Predictor of interest (X1)** | **YES / NO** |
| Histology | Adjustment Covariate (X2) | ADENOCARCINOMA / SQUAMOUS / OTHERS |
| RACE | Adj. Cov. (X3) | ORIENTAL / CAUCASIAN |
| SEX | Adj Cov. (X4) | FEMALE / MALE |
| RIN | Adj Cov. (X5) | [2,...,7.9] |

Patients with a stable disease status were excluded (n=31). The rationale was that by focusing on patients with the more marked response to therapy, the non-responder group would become more homogeneous.

For each probe-set i, the aim of the statistical test was to reject the hypothesis that the mean expression levels in patients with response to treatment and patients without response to treatment are equal, taking into account the other adjustment covariates listed in table 2. Formally, the null hypothesis of equality was tested against a two sided alternative. Under the null hypothesis, the distribution of the Wald-statistic for this test follows a Chi Square distribution with 64 degrees of freedom. The corresponding p-values are reported in table 3.

The choice of linear model was motivated by two reasons. Firstly, linear modeling is a versatile, well-characterized and robust approach that allows for adjustment of confound-ing variables when estimating the effect of the variable of interest. Secondly, given the sample size of 71, and the normalization and scaling of the data set, the normal distribution assumption was reasonable and justified.

The issue of multiple testing was dealt with by using a False Discovery Rate (FDR) criterion for identifying the list of differentially expressed genes (Benjamini et al., Journal of the Royal Statistical Society Series B-Methodological, 1995. 57(1): p. 289-300). Probe-sets with an FDR below the 0.3 threshold are declared significant. The 0.3 cut-off was cho-sen as a reasonable compromise between a rigorous correction for multiple testing with a stringent control of the risk of false positive and the risk of missing truly differential mark-ers. The identified marker gene is reported in Table 3.

Table 3: Markers based on comparing "Responders" to "Progressors". Responders were defined as patients with Best Response equal to "Partial Response" (PR). "Progres-sors" were defined as patients having best response equal to "Progressive Disease"(PD) or no assessment available.

Patients with no tumour assessment were included in the "Progressors" groups be-cause in the majority of cases, assessment was missing because of early withdrawal due to disease progression or death.

Column 1 is the Affymetrix identifier of the probe-set. Column 2 is the GenBank accession number of the corresponding gene sequence. Column 3 is the corresponding offi-cial gene name. Column 4 is the corresponding adjusted mean fold change in expression level between "responders" and "progressors", as estimated with the linear model. Column 5 is the p-vaiue for the test of difference in expression level between "responders" and "progressors" as derived from the linear model. Column 6 is the 95% confidence interval for the adjusted mean fold change in expression level.

| Affymetrix Probe Set ID | GenBank | Gene | Adjusted Mean Fold Change | P-value | CI 95% |
|---|---|---|---|---|---|
| 205194_at | NM_004577 | PSPH | 2.6 | 1.40E-05 | 1.8 , 3.7 |

For each probe-set, the assumption of homogeneity of variance was evaluated using Fligner-Killeen tests based on the model residuals. The analysis consisted of three steps:
Test all categorical variables for equality of residual variance between their levels
Note the variable V with the least p-value
If the least p-value is less than 0.001, re-fit the model allowing the different level of variables V to have a different variance.

### Further statistical analysis

For the selected candidate marker PSPH, the following additional analyses were performed in a validated environment by an independent statisticians :
- Univariate Cox Regression for PFS (Progression free survival) from Primary Affymetrix Analysis,
- Univariate Logistic Regression for Response from Primary Affymetrix Analysis, and
- Univariate Cox Regression for Survival from Primary Affymetrix Analysis.

The results of these analysis are presented below. They are consistent with the results of the primary analysis and confirm the choice of the selected marker.

Results: Univariate Cox Regression for PFS (Progression free survival) from Primary Affymetrix Analysis:

| **Gene** | **No. of patients** | **Hazard ratio** | **95 % CI for Hazard ratio** | **p-Value** |
|---|---|---|---|---|
| PSPH | 102 | 0.61 | 0.42; 0.88 | 0.0086 |

Results: Univariate Cox Regression for Response from Primary Affymetrix Analysis:

| **Gene** | **No. of patients** | **Odds ratio** | **95 % CI for Odds ratio** | **p-Value** |
|---|---|---|---|---|
| PSPH | 102 | 7.46 | 2.39; 23.32 | 0.0005 |

### qRT-PCR (quantitative Real-Time PCR)

cDNA was synthesized using SuperScriptTM III First-strand Synthesis SuperMix for qRT-PCR (Invitrogen, CA, USA) according to the manufacturer's instructions but without inclusion of an RNase H digest.

Quantitative PCR was performed using TaqMan® Gene Expression Assays on an ABI PRISM@ 7900HT Sequence Detection System according to the manufacturer's recommendations (Applied Biosystems, CA, USA). All assays were performed in triplicate.

The Gene Expression Assays used Hs00190154_m1 [PSPH], were chosen so that the primers and probes crossed exon boundaries or were within the Affymetrix Genechip® probe sequence of interest. Two house-keeping genes were included as endogenous con-trols: beta-2-microglobulin (B2M; Assay Hs99999907_m1) and hypoxanthinephosphoribo-syl transferase (HPRT; Assay Hs99999909_m1).

All runs included a calibrator sample (MVPTM total RNA from human adult lung; Stratagene, CA, USA) and a standard curve. Universal Human Reference total RNA (Stratagene, CA, USA) was used as template for PSPH standard curves. All samples were measured in triplicate.Relative quantification was performed using the -ΔCt method.

### Results

As reported above, Affymetrix Genechip® gene expression profiles were determined for 102 patients included in this study. Among these patients, qRT-PCR results were obtained for 75 (table 3). The demographics and clinical characteristics of the patients with qRT-PCR results were similar to those of the entire population (n=264) and of the patients with Genechip® gene expression profiles available.

**Table 3: Baseline characteristics: patients with qRT-PCR analyses (n=75)**

| **Characteristic** | |
|---|---|
| Age (median, range) | 62 (39-85) |

| Gender; n (%) | |
|---|---|
| Male | 19 (25) |
| Female | 56 (75) |

| ECOG performance status; n (%) | |
|---|---|
| 0 | 7 (9) |
| 1 | 45 (60) |
| 2 | 23 (31) |

| Histology; n (%) | |
|---|---|
| Adenocarcinoma | 27 (36) |
| Squamous-cell carcinoma | 34 (45) |
| Large-cell carcinoma | 2 (3) |
| Other | 12 (16) |

| Disease stage; n (%) | |
|---|---|
| IIIB | 22 (29) |
| IV | 53 (71) |

| Number of prior chemotherapy regimens; n (%) | |
|---|---|
| 0 | 19 (25) |
| 1 | 36 (48) |
| ≥2 | 20 (27) |

| Ethnicity; n (%) | |
|---|---|
| Caucasian | 51 (68) |
| Asian | 24 (32) |

| Smoking history; n (%) | |
|---|---|
| Never | 12 (16) |
| Current | 24 (32) |
| Former | 39 (52) |

Of the 75 patients with qRT-PCR results, 4 (5%) had partial response (PR), 23 (31%) had SD, 39 (52%) had PD, and 9 (12%) were not evaluable. These results were very similar to those observed in the entire study population (n=264).

### Correlation between Affymetrix Genechip® data, qRT-PCR data and clinical outcomes

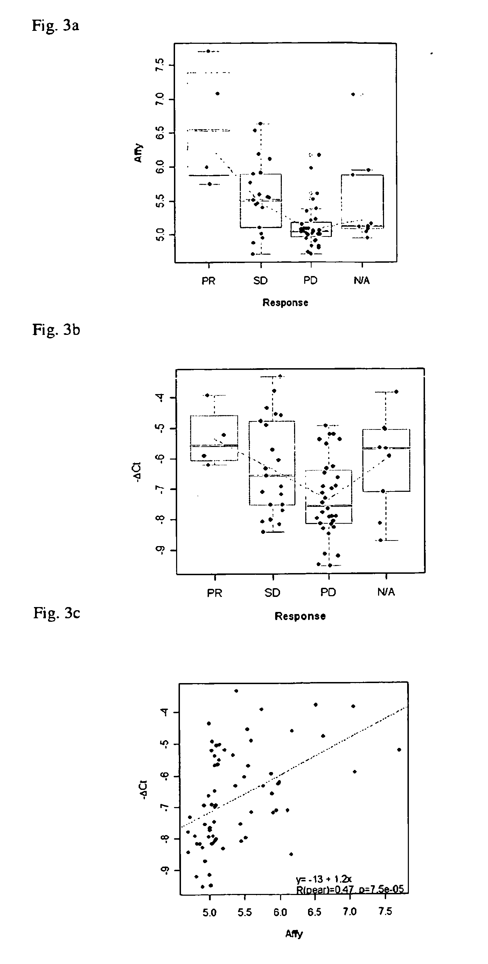

Figure 3 shows relative mRNA levels for PSPH in individual patients, as assessed by Affymetrix GeneChip® profiling and qRT-PCR. Fig. 3a shows expression levels versus clinical outcome for Genechip® profiling and Fig. 3b shows expression levels versus clinical outcome for qRT-PCR.

Figure 3c shows the correlation between Genechip® and qRT-PCR measurements for PSPH. The association between PSPH mRNA levels and clinical outcome with erlotinib observed in the Genechip® gene expression data was preserved in the qRT-PCR data.

### Identification of genes associated with response to erlotinib

Responders were defined as patients whose best response was partial response, while "progressors" were defined as patients having either progressive disease or for whom no assessment was made (in most cases as a result of early withdrawal due to disease progression or death). Thus in this model 6 "responders" were compared to 65 "progressors".

A linear model was fitted independently to each of the 16353 remaining probe-sets used in the analysis after removal of those probe-sets that were not present in any sample from the total 22283 on the HG-U133A microarray. A p-value was calculated for the difference in expression between response and non-response for each probe-set. A false discovery rate (FDR) of 0.3 was applied to correct for multiple testing. The marker identified from this analysis is shown in Table 3.

### Discussion

Targeting the Epidermal Growth Factor Receptor (EGFR) as a means of cancer therapy was proposed based on its ubiquitous aberrant expression in several epithelial cancers. EGFR is implicated in the pathogenesis and progression of many tumours including 40-80% of NSCLC tumours, as a result of activating mutations in the tyrosine kinase domain and / or its amplification. Upon activation, the receptor undergoes dimerization, resulting in phosphorylation of downstream targets with roles in cellular proliferation, metastasis, inhibition of apoptosis and neoangiogenesis.

Two major classes of EGFR inhibitors have been developed, monoclonal antibodies targeting the extracellular domain of the receptor, and small molecule tyrosine kinase inhibitors targeting the catalytic domain of the receptor. The latter include erlotinib which competes with ATP for the intracellular binding site.

It has emerged in recent years that several factors play a role in sensitivity to erlotinib including female gender, non-smoker status, Asian origin and adenocarcinoma histology; given that enhanced response rates are evident in such clinical subsets of patients, exten-sive efforts are ongoing to elucidate predictive molecular markers for patient stratification. Mutations in the EGFR, amplification of the EGFR gene locus and overexpression of EGFR on the protein level, have all been associated with response to varying degrees, though these are not the only molecular determinants of response.

By analyzing tissue samples with high-density oligonucleotide microarray technol-ogy, and applying statistical modeling to the data, we have been able to identify a gene whose expression level is predictive of response to erlotinib (comparison of PR versus PD) (Table 3). This gene is PSPH (2.6 fold upregulated; p = 0.000014).

Phosphoserine phosphatase (PSPH) is an intermediary enzyme in gluconeogenesis and amino acid biosynthesis, and is specifically responsible for the last step in L-serine formation. PSPH enzyme activity has been found to be higher in cancerous tissue than in normal lung tissue as well as in highly-differentiated lung tumours as compared to undif-ferentiated lung tumours or mesothelioma. On the mRNA transcript level PSPH has been evaluated as a marker for micrometastases in advanced gastric cancer and showed in-creased specificity in combination with a standard marker, carcinoembryonic antigen (CEA).

## Claims

1. An in vitro method of predicting the response of a NSCLC patient to treatment with erlotinib, comprising: determining an expression level of a gene PSPH in a tumour sample of a patient and comparing the expression level of the gene PSPH to a value representative of a gene PSPH expression level in tumours of a non responding patient population, wherein a higher expression level of the PSPH gene in the tumour sample of the patient is indicative for a patient who will respond to the treatment, wherein benefiting population was defined as either having objective response, or disease stabilization for 12 weeks.

2. The method of claim 1, wherein the expression level is determined by microarray technology.

3. The method of claim 1 or 2, wherein the marker gene shows between 1.8 and 3.7 or more fold higher expression in the tumour sample of the responding patient compared to the value representative of the gene PSPH expression level in tumours of a non responding patient population, wherein benefiting population was defined as either having objective response, or disease stabilization for 12 weeks.

## Patentansprüche

1. In-vitro-Verfahren zur Vorhersage des Ansprechens eines NSCLC-Patienten auf die Behandlung mit Erlotinib, umfassend: Bestimmung eines Expressionsspiegels eines PSPH-Gens in einer Tumorprobe eines Patienten und Vergleich des Expressionsspiegels des PSPH-Gens mit einem Wert, der repräsentativ für den PSPH-Gen-Expressionsspiegel in Tumoren einer nicht ansprechenden Patientenpopulation ist, wobei ein höherer Expressionsspiegel des PSPH-Gens in der Patienten-Tumorprobe einen Patienten anzeigt, der auf die Behandlung ansprechen wird, wobei die Nutzen ziehende Population entweder als eine mit objektivem Ansprechen oder mit einer Stabilisierung der Erkrankung für 12 Wochen definiert wurde.

2. Verfahren nach Anspruch 1, wobei der Expressionsspiegel durch Microarray-Technologie bestimmt wird.

3. Verfahren nach Anspruch 1 oder 2, wobei das Markergen eine zwischen 1,8-und 3,7-fache oder höhere Expression in der Tumorprobe des ansprechenden Patienten zeigt, verglichen mit dem Wert, der repräsentativ für den PSPH-Gen-Expressionsspiegel in Tumoren einer nicht ansprechenden Patientenpopulation ist, wobei die Nutzen ziehende Population entweder als eine mit objektivem Ansprechen oder mit einer Stabilisierung der Erkrankung für 12 Wochen definiert wurde.

## Revendications

1. Procédé in *vitro* de prédiction de la réponse d'un patient atteint de CBPNC (cancer bronchique non à petites cellules) au traitement avec l'erlotinib, comprenant: la détermination du niveau d'expression d'un gène PSPH dans un échantillon de tumeur du patient et la comparaison du niveau d'expression du gène PSPH à une valeur représentative du niveau d'expression du gène PSPH dans des tumeurs d'une population de patients non répondeurs, un niveau d'expression élevé du gène PSPH dans l'échantillon de tumeur du patient indiquant un patient qui répondra au traitement, où la population en profitant a été définie comme présentant soit une réponse objective, soit une stabilisation de la maladie pendant 12 semaines.

2. Procédé selon la revendication 1, dans lequel le niveau d'expression est déterminé par une technologie des puces à ADN.

3. Procédé selon la revendication 1 ou 2, dans lequel le gène marqueur présente une expression entre 1,8 et au moins 3,7 fois plus élevée dans l'échantillon de tumeur du patient répondeur par comparaison à la valeur représentative du niveau d'expression du gène PSPH dans les tumeurs d'une population de patients non répondeurs, où la population en profitant a été définie comme présentant soit une réponse objective, soit une stabilisation de la maladie pendant 12 semaines.


### SEQUENCE LISTING

<110> F. Hofffmann-La Roche AG
<120> Predictive marker for EGFR inhibitor treatment
<130> 24412WO
<150> 07114312.7
   <151> 2007-08-14
<150> 08156511.1
   <151> 2008-05-20
<160> 1
<170> PatentIn version 3.4
<210> 1
   <211> 2142
   <212> DNA
   <213> Homo sapiens
<400> 1
   ggcgttggag ctctttgggg cccagctttg cggacccggg agctcgggac gcaggcgggg 60
   cttgtgctcc gcgggggcag ggcgtagggt gggcctccta cctcccctga tctcgcggtt 120
   tgttccgttt cattggagct tcccggaccg tgtgctcgac ggtgccctag gtgccgtggg 180
   gccacacgcg agtctgataa gcaccctccc ccggaatcat gcggtgctgt gaggcctagc 240
   gaagatgaag atagaatgca aggtagaaag tgctggatac ctttagaaag ctgcaggact 300
   ggtgcgatgg gagttgagac gtaagaacct gcccgtccgt agggctctgg atgctgctga 360
   ggcccgaggc ccctatggca gatttgaaaa ttcacccttg tagagtcatt cctgcctttg 420
   agcggactcc cttttaagca gatctcaaga gagcgttcgg tggaggccct gggtctgcac 480
   agctcacctc cctgggaact gctcgcccga gcgtcggagc cggcgctggc cccctgcagc 540
   cggaaggttg cagccgcagg agccccggag gcccaggaca cagggctctt gctcttgcag 600
   aatccacagg tctttcttga ggaaatctgt agacagaact ttgtgctgcg tttttatcta 660
   gggaaggaac agaagagtgt cgtctcctag aaatctagca ctggagaaac gaggaaaatt 720
   cttccagcga tggtctccca ctcagagctg aggaagcttt tctactcagc agatgctgtg 780
   tgttttgatg ttgacagcac ggtcatcaga gaagaaggaa tcgatgagct agccaaaatc 840
   tgtggcgttg aggacgcggt gtcagaaatg acacggcgag ccatgggcgg ggcagtgcct 900
   ttcaaagctg ctctcacaga gcgcttagcc ctcatccagc cctccaggga gcaggtgcag 960
   agactcatag cagagcaacc cccacacctg acccccggca taagggagct ggtaagtcgc 1020
   ctacaggagc gaaatgttca ggttttccta atatctggtg gctttaggag tattgtagag 1080
   catgttgctt caaagctcaa tatcccagca accaatgtat ttgccaatag gctgaaattc 1140
   tactttaacg gtgaatatgc aggttttgat gagacgcagc caacagctga atctggtgga 1200
   aaaggaaaag tgattaaact tttaaaggaa aaatttcatt ttaagaaaat aatcatgatt 1260
   ggagatggtg ccacagatat ggaagcctgt cctcctgctg atgctttcat tggatttgga 1320
   ggaaatgtga tcaggcaaca agtcaaggat aacgccaaat ggtatatcac tgattttgta   1380
   gagctgctgg gagaactgga agaataacat ccattgtcgt acagctccaa acaacttcag   1440
   atgaattttt acaagttata cagattgata ctgtttgctt acagttgcct attacaactt   1500
   gctatagaaa gttggtacaa atgatctgta ctttaaacta cagttaggaa tcctagaaga   1560
   ttgctttttt ttttttttta actgtagttc cagtattata tgatgactat tgatttcctg   1620
   gagaggtttt tttttttttt gagacagaat cttgctctgt tgcccaggct ggagtgcagt   1680
   ggcgcggtct cggctcactg caagctctgc ctcccaggtt cacgccattc tcctgcctca   1740
   gcctcccgag tagctgggac tacaggcacc cgccaccaca tccggctaat tttttgtatt   1800
   tttagtagag acggggtttg accgtgttag ccaggatggt cttgatctcc tgaccttgtg   1860
   atccgcctgc ctcagcctcc caaagtgctg ggattacagg cttgggccac cgcgcccagc   1920
   caatgtccta gagagttttg tgatctgaat tctttatgta tatttgtagc tatatttcat   1980
   acaaagtgct ttaagtgtgg agagtcaatt aaacaccttt actcttagaa atacggattc   2040
   ggcagccttc agtgaatatt ggtttctctt tggtatgtca ataaaagttt atccgtatgt   2100
   cagaacggat ttgtggaaaa aaaaaaaaaa aaaaaaaaaa aa                      2142